# FASCICULE DE BREVET EUROPEEN

(11) **EP 0 580 502 B1**
(45) Date de publication et mention de la délivrance du brevet: **02.09.1998**
(21) Numéro de dépôt: 93401875.5
(22) Date de dépôt: 21.07.1993
(51) Int. Cl.: C07D 209/34, A61K 31/40, C07D 401/04

(54) **3-(hydroxybenzylidényl)-indoline-2-ones, et les compositions pharmaceutiques qui les contiennent**
3-(Hydroxybenzylidenyl)-Indolin-2-one und diese enthaltende Arzneimittel
3-(Hydroxybenzylidenyl)-indoline-2-ones and pharmaceutical compositions containing them

(30) Priorité: 21.07.1992 FR 9208951
(43) Date de publication de la demande: 26.01.1994
(73) Titulaire: ADIR ET COMPAGNIE, 92415 Courbevoie Cédex (FR)
(72) Inventeur: Le Baut, Guillaume, F-44230 Saint-Sebastien sur Loire (FR); Nourisson, Marie Renée, F-44240 La Chapelle sur Erdre (FR); Renaud de la Faverie, Jean-François, F-78150 Le Chesnay (FR); Bizot-Espiard, Jean-Guy, F-75015 Paris (FR); Caignard, Daniel-Henri, F-75015 Paris (FR); Renard, Pierre, F-78000 Versailles (FR); Adam, Gérard, F-78600 Le Mesnil Le Roi (FR)

(56) Documents cités:
- EP-A- 429 685
- WO-A-92/07830
- US-A- 5 124 347

## Description

La présente invention concerne de nouvelles 3-(hydroxybenzylidényl)-indoline-2-ones, leur procédé de préparation et les compositions pharmaceutiques qui les contiennent.

Les composés de formule (a), dans laquelle R₁ représente un atome d'hydrogène ou un groupement méthyle, ont été décrits dans la publication de ZHUNGIETU et al. (Khim. Geterotsikl. Soedin., 1973, (1) : pages 40-44) comme produits de réaction d'indoles et d'hydroxyindole avec certains aldéhydes mais aucune activité pharmacologique n'a été mentionnée. 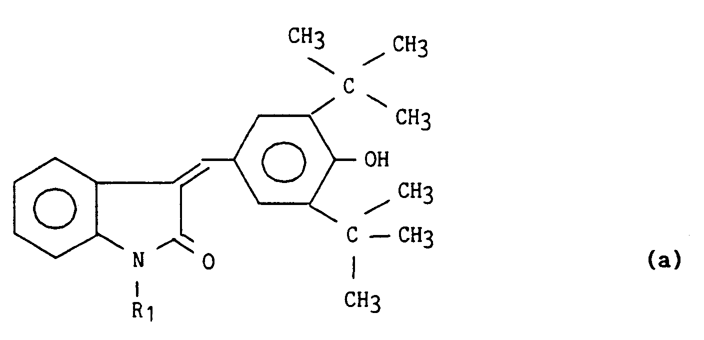 On connait également de l'art antérieur les composés de formule (b) : 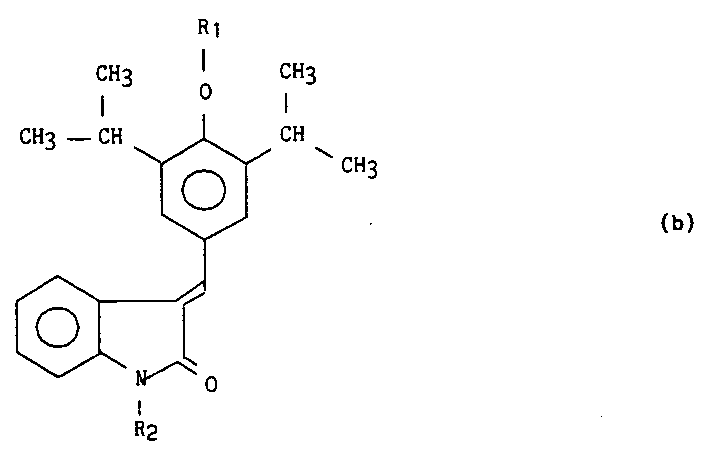 dans laquelle R₁ est un atome d'hydrogène ou un radical benzyle et R₂ représente un atome d'hydrogène, un radical formyle, (C₁-C₃)acyle, ou phényle.

Ces composés de formule (b) ont été présentés dans la demande JP 62029570, comme agents anti-allergiques et inhibiteurs de tyrosine kinase.

La demande EP 429 685 décrit des composés indolinoniques acylés en position 1, utiles dans le traitement des démences séniles. Le brevet WO 9207830 décrit des indolinones inhibitrices de GRP (Gastrin Released Peptide) utiles notamment dans le traitement du cancer du poumon à petites cellules et des psychoses.

Le brevet US 5,124,347 décrit des composés de structure 3(3,5-di-tert-butyl-4-hydroxybenzylidényl)-indolinonique comme agents anti-inflammatoires.

La demanderesse a découvert de nouvelles 3-(hydroxy-benzylidényl)-indolin-2-ones qui possédent des propriétés pharmacologiques très intéressantes.

En effet, les nouvelles 3-(hydroxy-benzylidényl)-indolin-2-ones de l'invention possèdent des propriétés antioxydantes très importantes, que ne possèdent pas les composés du brevet 5,124,347.

Plus particulièrement, la présente invention concerne les composés de formule (I) : 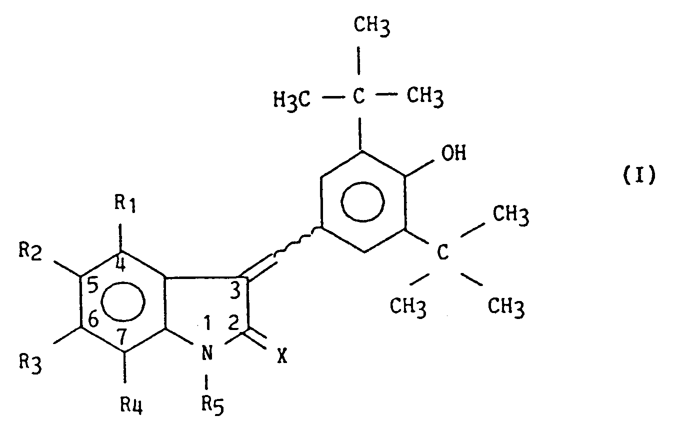 dans laquelle :
X représente un atome de soufre ou d'oxygène,
R₁, R₂, R₃, R₄, identiques ou différents, représentent chacun indépendamment l'un de l'autre :
   - un atome d'hydrogène,
   - ou un radical choisi parmi :
      . halogène,
      . hydroxyle,
      . un groupement -E₁ ou 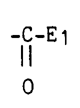 où E₁ représente un groupement alkyle inférieur, alcényle inférieur, alcynyle inférieur, ou alkoxy inférieur, le groupement E₁ pouvant être non substitué ou substitué par un ou plusieurs radicaux choisis parmi halogène, alkyl inférieur, alkoxy inférieur, alkylamino inférieur, et dialkylamino inférieur,
      . et un groupement -(CH₂)ₙ-E₂, -O-(CH₂)ₙE₂, 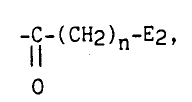 ou 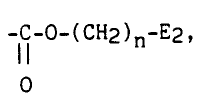 où n représente 0 ou un entier de 1 à 4 et où E₂ est choisi parmi :
         - phényle ou naphtyle, non substitués ou substitués par un ou plusieurs radicaux choisi parmi halogène, hydroxyle, alkyle inférieur, alkoxy inférieur, et trifluorométhyle,
         - et cycloalkyle de 3 à 8 atomes de carbone, non substitué ou substitué par un ou plusieurs radicaux choisis parmi halogène, oxo, alkyle inférieur, et alkoxy inférieur,
R₅ représente un radical choisi parmi :
   - hydroxyle,
   - un groupement -E₃ où E₃ représente un groupement acyle inférieur, alkoxy inférieur, ou un groupement -(CH₂)ₙ-CO-R₆ dans lequel n représente 0 ou un nombre entier de 1 à 6 et où R₆ représente un radical hydroxyle ou alkoxy inférieur,
      le groupement E₃ pouvant être non substitué ou substitué par un ou plusieurs radicaux choisis parmi halogène, hydroxyle, alkyle inférieur, et alkoxy inférieur,
   - un groupement phényle ou -O-(CH₂)ₘ-phényle, où m représente 0 ou un nombre entier de 1 à 4, le noyau phényle pouvant être non substitué ou substitué par un ou plusieurs radicaux choisis parmi halogène, hydroxyle, alkyle inférieur, alkoxy inférieur, et trifluorométhyle,
   - un groupement pyridyle ou -O-(CH₂)ₘ-pyridyle où m est tel que défini précédemment, le noyau pyridyle pouvant être non substitué ou substitué par un ou plusieurs radicaux choisis parmi halogène, hydroxyle, alkyle inférieur, et alkoxy inférieur,
   - un groupement -(CH₂)ₘ-E₄, -O-(CH₂)ₘ-E₄, ou 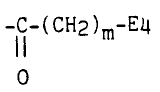 où m représente 0 ou un nombre entier de 1 à 4 et E₄ est un radical choisi parmi naphtyle, pyrimidinyle, thiényle, furyle, et pyrrolyle, E₄ étant non substitué ou substitué par un ou plusieurs radicaux choisis parmi halogène, hydroxyle, alkyle inférieur, alkoxy inférieur, et trifluorométhyle,
   - et un goupement cycloalkyle ou cycloalkylalkyle inférieur, où le radical cycloalkyle contient de 3 à 8 atomes de carbone et est non substitué ou substitué par un ou plusieurs radicaux choisis parmi halogène, oxo, alkyle infér.ieur, et alkoxy inférieur,
      étant entendu que, sauf précision contraire :
   - les termes "alkyle inférieur", "alkoxy inférieur", et "acyle inférieur" désignent des groupements linéaires ou ramifiés contenant de 1 à 6 atomes de carbone,
   - et les termes "alcényle inférieur" et "alcynyle inférieur" représentent des groupements insaturés de 2 à 6 atomes de carbone,
      leurs isomères Z et E, leurs isomères optiques sous forme pure, ou sous forme de mélange, et leurs sels d'addition à un acide ou une base pharmaceutiquement acceptable.

Plus particulièrement, l'invention s'étend aux composés de formule (I) pour lesquels R₅ représente un groupement alcoxy inférieur et aux composés de formule (I) pour lesquels R₁, R₂, R₃ et R₄ sont choisis parmi hydrogène, halogène, hydroxyle, alkyle inférieur, alcoxy inférieur, et trifluorométhyle, leurs isomères Z et E, leurs isomères optiques, sous forme pure ou sous forme de mélange, et leurs sels d'addition à un acide ou à une base pharmaceutiquement acceptable.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour salifier les composés utilisés selon l'invention, on peut citer, à titre d'exemples et de façon non limitative la soude, la potasse, la triéthylamine, la diéthylamine, l'éthanolamine, l'arginine, la lysine, et la diéthanolamine.

La présente invention s'étend également au procédé de préparation des composés de formule (I) caractérisé en ce que
on fait réagir un composé de formule (II) 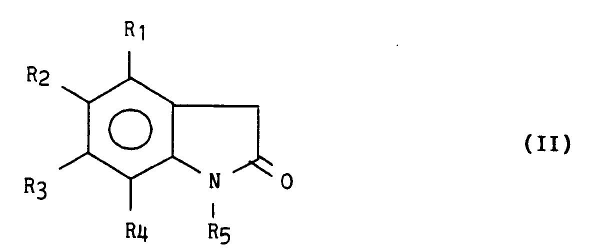 dans laquelle R₁, R₂, R₃, R₄, et R₅ sont tels que définis dans la formule (I)
avec un composé de formule (III) 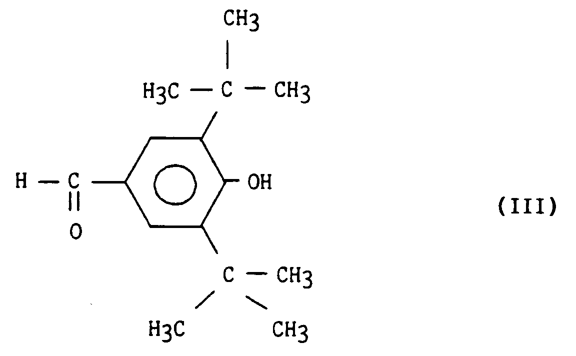 pour obtenir un composé de formule (I/a) 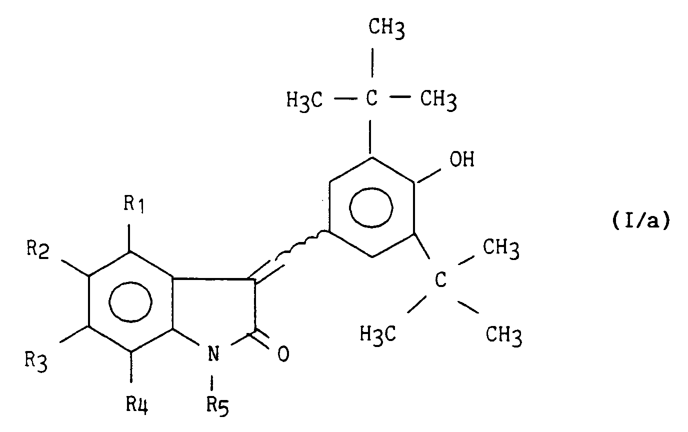 dans laquelle R₁, R₂, R₃, R₄ et R₅ sont tels que définis dans la formule (I),
qui peut être soumis au réactif de Lawesson pour obtenir un composé de formule (I/b) 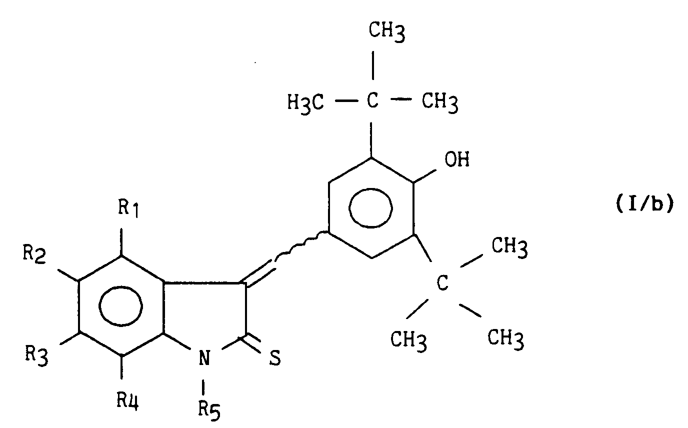 dans laquelle R₁, R₂, R₃, R₄ et R₅ sont tels que définis précédemment,
les composés de formule (I/a) et (I/b) formant l'ensemble des composés de formule (I),
les composés de formule (I) pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères Z et E ou en leurs isomères optiques,
- ou salifiés par un acide ou une base pharmaceutiquement acceptable.

Les matières premières utilisées dans le procédé précédemment décrit sont soit commerciales, soit aisément accessibles, à l'homme de l'art selon des procédés connus dans la littérature ou proposés lors des exemples de préparations décrits ci-après dans cette demande.

Les composés de la présente invention possèdent des propriétés antioxydantes très importantes. Les études pharmacologiques ont notamment montré que les composés de la présente invention étaient doués d'activités protectrices remarquables dans le cadre des processus de peroxydations des lipides cellulaires et des lipoprotéines de faible densité (LDL). Un tel niveau d'activité protectrice n'est pas retrouvé pour les composés du brevet US 5,124,347.

Par ailleurs, les composés de la présente invention présentent la particularité d'avoir un puissant effet inhibiteur sur la biosynthèse des prostanoïdes, très supérieur aux composés du brevet US 5,124,347. Ils possèdent également un important pouvoir antiagrégant plaquettaire.
Les activités pharmacologiques des composés de l'invention sont également très supérieures à celles des composés antioxydants de l'art antérieur, notamment à celle du probucol, composé commercialisé connu pour son pouvoir antioxydant et utilisé en thérapeutique.
On peut donc attendre des composés de l'invention, qui présentent à la fois des propriétés inhibitrices de la peroxydation lipidique, de la biosynthèse des prostanoïdes, et de l'agrégation plaquettaire, une action particulièrement nouvelle et bénéfique dans les affections où interviennent une peroxydation des lipides membranaires, un dérèglement de la synthèse des prostanoïdes ou des troubles de l'agrégation plaquettaire.

Les composés de formule (I) peuvent ainsi être utilisés pour l'obtention de médicaments utiles dans le traitement ou la prévention des affections dues ou reliées à des phénomènes de peroxydation, à des dérèglements de la synthèse des prostanoïdes ou à des troubles de l'agrégation plaquettaire, dans le traitement des désordres ischémiques, des maladies inflammatoires, de la douleur, des maladies métaboliques, de l'athérome, de l'artériosclérose, des maladies respiratoires, de l'asthme, de l'emphysème, des maladies d'origine immunologique, du psiorasis, du lupus érythémateux, des réactions allergiques, du vieillissement cérébral ou périphérique, et dans la prévention et le traitement des dommages dus aux traumatismes chirurgicaux et à la reperfusion d'organes.

La présente invention a également pour objet les compositions pharmaceutiques contenant au moins un composé de formule (I), ou un de ses sels d'addition à un acide ou à une base pharmaceutiquement acceptable, en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les capsules, les suppositoires, les crèmes, pommades, et les gels dermiques.

La posologie utile varie selon l'âge et le poids du patient, la voie d'administration, la nature de l'affection et des traitements éventuellement associés et s'échelonne entre 1 mg et 200 mg (de préférence de 1 à 50 mg, particulièrement de 1 à 20 mg, par exemple de 10 à 20 mg) par 24 heures en 1 à 2 prises.

Les préparations suivantes décrites sont utiles dans la synthèse des composés de l'invention. Elles ne font pas partie de l'invention.

Les composés des préparations suivantes sont synthésés suivant des procédés connus de l'homme de l'art développés notamment dans Héterocyclic compounds, vol. 3, John Wiley and Sons, Inc, New York pp 129-146, Julian P.L. et al.

### Préparation 1 : 1-METHOXY-INDOLIN-2-ONE

### STADE A : N-(METHOXY)-PHENYLACETAMIDE

Introduire dans un ballon 6,8 g (50 mmoles) d'acide phénylacétique acide et 4,6 g (55 mmoles) de chlorhydrate de méthoxylamine, 5,55 g (55 mmoles) de triéthylamine et enfin 10,83 g (52,5 mmoles) de N,N-dicyclohexylcarbodimide. Mettre en solution dans 150 cm³ de dichlorométhane. Mettre sous agitation pendant 16 heures à température ambiante. Eliminer le précipité par filtration et le laver par le dichlorométhane. Traiter le filtrat par 100 cm³ de HCl à 3 % puis par 100 cm³ d'eau et enfin par 100 cm³ de NaHCO3 à 5%.
Purifier éventuellement par passage sur colonne de silice en éluant par du dichlorométhane ou recristalliser dans l'éther isopropylique.
Recueillir 5,28 g de cristaux blancs (63,9 %).
Point de fusion : 68°C.
Caractéristiques spectrales :

| | | |
|---|---|---|
| infrarouge : ν cm⁻¹ | ν C = O | 1760, 1735 |
| | ν O-CH₃ | 2890 |
| | ν C = C | 1620, 1615, 1610, 1595 |

### STADE B : N-(METHOXY)-N-(CHLORO)PHENYLACETAMIDE

Dissoudre 1,65 g (10 mmoles) du N-(méthoxy)-phénylacétamide dans 30 cm³ de chloroforme sec. Placer dans un bain de glace et maintenir à une température de 0°C. Verser ensuite progressivement à l'aide d'une ampoule 1,30 g (1,36 cm³, 12 mmoles) d'hypochlorite de tertiobutyle préalablement dissous dans 20 cm³ de chloroforme.
Agiter à 0°C pendant 30 mn. Vérifier par chromatographie sur couche mince la fin de la réaction. Evaporer le chloroforme sous vide à 35°C.
Maintenir du papier aluminium sur le ballon pendant cette opération.
Si un passage sur colonne est souhaité, protéger la colonne de la lumière vive. Eluer par le dichlorométhane.
Recueillir 1,35 g de produit pur liquide, légèrement coloré (67,6 %).
Point de fusion : 68°C

### STADE C : 1-METHOXY-INDOLIN-2-ONE

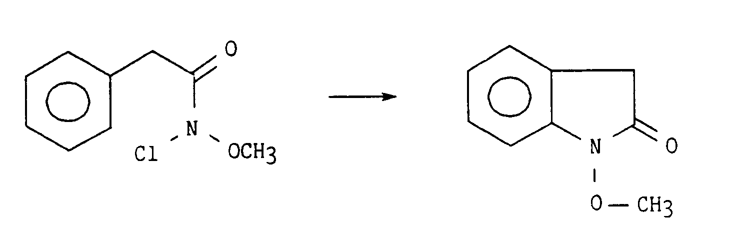

Dissoudre 1,35 g (20 mmoles) de N-benzylcarbonyl N-chloro N-méthoxyamine dans 20 cm³ d'acide trifluoroacétique. Placer dans un bain de glace pour refroidir. Puis ajouter une solution de carbonate d'argent (3,7 g, 20 mmoles) dans 3 cm³ d'acide trifluoroacétique. Mettre sous agitation magnétique 30 minutes jusqu'à ce que la réaction soit complète. Evaporer le solvant sous vide au dessous de 35°C.

Placer à nouveau le ballon dans un bain de glace et sous agitation magnétique. Ajouter une solution à 5 % de carbonate de sodium (environ 100 cm³). Filtrer le précipité formé et le laver avec du dichlorométhane. Extraire la phase aqueuse (filtrat) à l'aide du dichlorométhane. Puis traiter par une solution de chlorure de sodium. Sécher la phase organique sur Na₂SO₄. Filtrer, évaporer et purifier, si nécessaire, par passage sur colonne de gel de silice en éluant par le dichlorométhane. Recueillir 1,88 g de produit pur correspondant au composé du titre.
Rendement : 57,6 %
Point de fusion : 83°C
Caractéristiques spectrales :

| | | |
|---|---|---|
| infrarouge : ν en cm⁻¹ | ν C = O | 1675 |
| | ν O-CH₃ | 2900 |
| | ν C = C | 1650, 1645 |

### Préparations 2 à 17 :

En procédant comme dans la préparation 1 mais en remplaçant, au stade C, la N-benzylcarbonyl N-chloro N-méthoxyamine par la N-benzylcarbonyl N-chloro-N-méthoxyamine convenablement substituée sur le benzyle, on obtient les composés des préparations suivantes :
- **Préparation 2**: : **1,5,6-TRIMETHOXY-INDOLIN-2-ONE**
- Point de fusion: : 84 - 84,5°C
- **Préparation 3**: : **6-CHLORO-1-METHOXY-INDOLINE-2-ONE**
- Point de fusion: : 114-116°C
- Solvant: : ETHER ETHYLIQUE
- **Préparation 4**: : **6-BROMO-1-METHOXY-INDOLIN-2-ONE**
- Point de fusion: : 116-117°C
- Solvant: : ETHER ETHYLIQUE
- **Préparation 5**: : **1-METHOXY-6-METHYL-INDOLIN-2-ONE**
- Point de fusion: : 64 - 65°C
- Solvant: : ETHER ETHYLIQUE/HEXANE
- **Préparation 6**: : **1-METHOXY-5-BENZYLOXY-INDOLIN-2-ONE**
- Point de fusion: : 188 - 189°C
- **Préparation 7**: : **4-IODO-1-METHOXY-INDOLIN-2-ONE**
- Point de fusion: : 127 - 128°C
- Solvant: : ETHER ETHYLIQUE
- **Préparation 8**: : **4-FLUORO-1-METHOXY-INDOLIN-2-ONE**
- Point de fusion: : 106-107°C
- Solvant: : ETHER ETHYLIQUE
- **Préparation 9**: : **5-CHLORO-1-METHOXY-INDOLIN-2-ONE**
- **Préparation 10**: : **1,5-DIMETHOXY-INDOLIN-2-ONE**
- **Préparation 11**: : **1-METHOXY-4-TRIFLUOROMETHYL-INDOLIN-2-ONE**
- **Préparation 12**: : **1-METHOXY-6-TRIFLUOROMETHYL-INDOLIN-2-ONE**
- **Préparation 13**: : **1-METHOXY-5-METHYL-INDOLIN-2-ONE**
- **Préparation 14**: : **5,6-DIMETHYL-1-METHOXY-INDOLIN-2-ONE**
- **Préparation 15**: : **5-ETHYL-1-METHOXY-INDOLIN-2-ONE**
- **Préparation 16**: : **6-ISOPROPYL-1-METHOXY-INDOLIN-2-ONE**
- **Préparation 17**: : **5-CYCLOPROPYLMETHYL-1-METHOXY-INDOLIN-2-ONE**
- **Préparation 18**: : **5-CYCLOPROPYLOXY-1-METHOXY-INDOLIN-2-ONE**
- **Préparation 19**: : **1-(PYRID-2-YL)-INDOLIN-2-ONE**

Dans un ballon de 500 cm³, introduire 10,50 g (90 mmoles) d'indole, 375 cm³ de diméthyl formamide anhydre, 17,07 g (108 mmoles) de 2-bromopyridine, 41,25 g de cuivre en poudre et 90 g de carbonate de potassium.

Chauffer au reflux sous agitation pendant 24 heures, la réaction est suivie sur chromatographie couche mince dans le chlorure de méthylène. Filtrer le milieu réactionnel pour éliminer le carbonate et le cuivre. Evaporer le diméthyl formamide. On obtient un produit pâteux brun.

Purifier par 2 passages successifs sur colonne de gel de silice en éluant par un mélange éther de pétrole/acétate d'éthyle (9/1) puis par du chlorure de méthylène. Recueillir 16,8 g de liquide jaune pâle (96 %) correspondant au 1-(pyrid-2-yl)-indole.

Mettre le composé ainsi obtenu lors de l'étape précédente en solution dans du chlorure de méthylène. Le mettre sous atmosphère d'azote et y ajouter en 4 fractions, à 30 mn d'intervalle, 103 g (75,3 mmoles) de N-chloro succinimide. Laisser sous agitation pendant 1 heure à 15-20°C sous atmosphère d'azote. Puis verser le milieu réactionnel sur de l'eau. Extraire par du chlorure de méthylène. Sécher sur Na₂ SO₄. Evaporer pour obtenir le 3-chloro-1-(pyrid-2-yl)-indole.

Dissoudre le 3-chloro-1-(pyrid-2-yl)-indole dans 1 500 cm³ de 2-méthoxyéthanol et chauffer à 100°C sous agitation. Ajouter 1 100 cm³ d'acide phosphorique à 70 % en filet. Poursuivre le chauffage pendant 6 heures, puis traiter le milieu réactionnel par du charbon au reflux pendant 15 mn. Filtrer. Chauffer à 70°C en ajoutant 2 000 cm³ d'eau. Laisser refroidir à 5°C pendant 12 heures le produit précipité. Filtrer. Sécher.
- **Préparation 20**: **: 1-(4,6-DIMETHYLPYRID-2-YL)-INDOLIN-2-ONE**

En procédant comme dans la préparation 26 mais en utilisant la pyridine convenablement substituée, on obtient le composé du titre :
- **Préparation 21**: **: 1-(2,6-DICHLOROPHENYL)-INDOLIN-2-ONE**

Après réaction, durant deux heures, de l'acide 2-chlorophénylacétique et de la 2,6-dichloro-aniline en présence de carbonate de potassium, on obtient le composé du titre.
- **Préparation 22**: **: 1-PHENYL-INDOLIN-2-ONE**

En procédant comme dans la préparation 21, mais en remplaçant la 2,6-dichloro-aniline par l'aniline, on obtient le composé du titre.

### Préparations 23 à 27 :

En suivant les procédés exposés précédemment, on obtient les composés des préparations suivantes :
- **Préparation 23**: **: 1-ACETYL-INDOLIN-2-ONE**
- **Préparation 24**: **: 1-[3-(3,5-DI-TERT-BUTYL-4-HYDROXYBENZYLIDENYL)-INDOLIN-** **2-ONE]ACETATE D'ETHYLE**
- **Préparation 25**: **: ACIDE 1-[3-(3,5-DI-TERT-BUTYL-4-HYDROXYBENZYLIDENYL)-INDOLIN-2-ONE]ACETIQUE**
- **Préparation 26**: **: 5-CHLORO-1-(PYRYD-2-YL)-INDOLIN-2-ONE**
- **Préparation 27**: **: 6-CHLORO-1-(2,6-DICHLOROPHENYL)-INDOLIN-2-ONE**
- **Préparation 28**: **: 1-(6-METHOXYPYRID-2-YL)-INDOLIN-2-ONE**

Les exemples qui suivent illustrent l'invention et ne la limitent en aucune façon.
- **EXEMPLE 1**: **: 6-BROMO-3-(3,5-DI-TERT-BUTYL-4-HYDROXYBENZYLIDENYL)-1-** **METHOXY-INDOLIN-2-ONE**

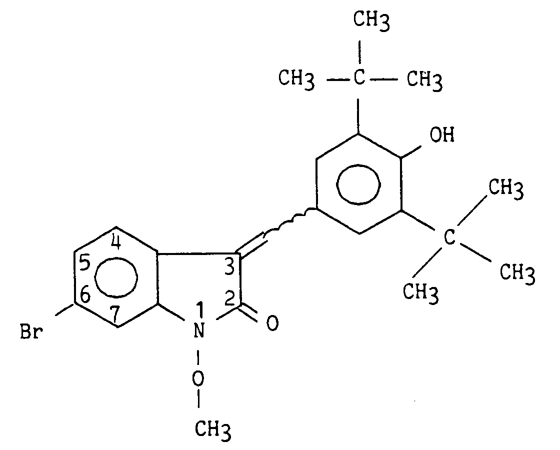

Dissoudre 2 g (8,3 mmoles) de 6-bromo-1-méthoxy-indolin-2-one dans 25 cm³ d'éthanol absolu. Ajouter 2,02 g (8,3 mmoles) de 3,5-di-tert-butyl-4-hydroxybenzaldéhyde, puis 0,5 à 1 cm³ de pipéridine. Mettre sous reflux léger. Suivre la réaction sur chromatographie couche mince et arrêter le chauffage après 3 h de reflux. Evaporer le solvant et purifier le produit obtenu par passage sur colonne de gel de silice en éluant par du dichlorométhane. Recueillir d'abord l'aldéhyde qui n'a pas réagi puis le composé du titre.
Rendement : 47,4 %
Point de fusion : 132°C
Solvant de recristallisation : Ether diisopropylique/éthanol

- **EXEMPLE 2**: **: 6-CHLORO-3-(3,5-DI-TERT-BUTYL-4-HYDROXYBENZYLIDENYL)-1-** **METHOXY-INDOLIN-2-ONE**

En procédant comme dans l'exemple 1, mais en remplaçant la 6-bromo-1-méthoxy-indolin-2-one par la 6-chloro-1-méthoxy-indolin-2-one, on obtient le composé du titre.
Point de fusion : 165°C

- **EXEMPLE 3**: **: 3-(3,5-DI-TERT-BUTYL-4-HYDROXYBENZYLIDENYL)-1-(2,6**-**DICHLOROPHENYL)-INDOLIN-2-ONE**

En procédant comme dans l'exemple 1, mais en remplaçant la 6-bromo-1-méthoxy-indolin-2-one par la 1-(2,6-dichlorophényl)-indolin-2-one, on obtient le composé du titre.
Rendement : 80 %
Point de fusion : 159°C
Solvant de recristallisation : chlorure de méthylène

- **EXEMPLE 4**: **: 3-(3,5-DI-TERT-BUTYL-4-HYDROXYBENZYLIDENYL)-1-PHENYL-INDOLIN-2-ONE**

En procédant comme dans l'exemple 1, mais en remplaçant la 6-bromo-1-méthoxy-indolin-2-one par la 1-phényl-indolin-2-one, on obtient le composé du titre.

### EXEMPLES 5 A 19 :

En procédant comme dans l'exemple 1 mais en remplaçant la 6-bromo-1-méthoxy-indolin-2-one par la 1-méthoxy-indolin-2-one convenablement substitué, on obtient les composés des exemples suivants :
- **EXEMPLE 5**: **: 3-(3,5-DI-TERT-BUTYL-4-HYDROXYBENZYLIDENYL)-1,5,6-TRIMETHOXY-INDOLIN-2-ONE**
- **EXEMPLE 6**: **: 3-(3,5-DI-TERT-BUTYL-4-HYDROXYBENZYLIDENYL)-1-METHOXY-6-** **METHYL-INDOLIN-2-ONE**
- **EXEMPLE 7**: **: 3-(3,5-DI-TERT-BUTYL-4-HYDROXYBENZYLIDENYL)-1-METHOXY-5-** **BENZYLOXY-INDOLIN-2-ONE**
- **EXEMPLE 8**: **: 3-(3,5-DI-TERT-BUTYL-4-HYDROXYBENZYLIDENYL)-4-IODO-1-** **METHOXY-INDOLIN-2-ONE**
- **EXEMPLE 9**: **: 3-(3,5-DI-TERT-BUTYL-4-HYDROXYBENZYLIDENYL)-4-FLUORO-1-** **METHOXY-INDOLIN-2-ONE**
- **EXEMPLE 10**: **: 3-(3,5-DI-TERT-BUTYL-4-HYDROXYBENZYLIDENYL)-5-CHLORO-1-METHOXY-INDOLIN-2-ONE**
- **EXEMPLE 11**: **: 3-(3.5-DI-TERT-BUTYL-4-HYDROXYBENZYLIDENYL)-1,5-DIMETHOXY-INDOLIN-2-ONE**
- **EXEMPLE 12**: **: 3-(3,5-DI-TERT-BUTYL-4-HDYROXYBENZYLIDENYL)-1-METHOXY-4-TRIFLUOROMETHYL-INDOLIN-2-ONE**
- **EXEMPLE 13**: **: 3-(3,5-DI-TERT-BUTYL-4-HYDROXYBENZYLIDENYL)-1-METHOXY-6-TRIFLUOROMETHYL-INDOLIN-2-ONE**
- **EXEMPLE 14**: **: 3-(3,5-DI-TERT-BUTYL-4-HYDROXYBENZYLIDENYL)-1-METHOXY-5-METHYL-INDOLIN-2-ONE**
- **EXEMPLE 15**: **: 3-(3,5-DI-TERT-BUTYL-4-HYDROXYBENZYLIDENYL)-1-METHOXY-5-ETHYL-INDOLIN-2-ONE**
- **EXEMPLE 16**: **: 3-(3,5-DI-TERT-BUTYL-4-HYDROXYBENZYLIDENYL)-1-METHOXY-5,6-DIMETHYL-INDOLIN-2-ONE**
- **EXEMPLE 17**: **: 3-(3,5-DI-TERT-BUTYL-4-HYDROXYBENZYLIDENYL)-6-ISOPROPYL-1-METHOXY-INDOLIN-2-ONE**
- **EXEMPLE 18**: **: 3-(3,5-DI-TERT-BUTYL-4-HYDROXYBENZYLIDENYL)-5-CYCLOPROPYLMETHYL-1-METHOXY-INDOLIN-2-ONE**
- **EXEMPLE 19**: **: 3-(3,5-DI-TERT-BUTYL-4-HYDROXYBENZYLIDENYL)-5-CYCLOPROPYLOXY-1-METHOXY-INDOLIN-2-ONE**
- **EXEMPLE 20**: **: 3-(3,5-DI-TERT-BUTYL-4-HYDROXYBENZYLIDENYL)-1-(PYRYD-2-YL)-INDOLIN-2-ONE**

Dans un ballon de 100 cm³, introduire 2 g (9,5 mmoles) de 1-(pyrid-2-yl)indolin-2-one, 40 cm³ de benzène anhydre, 3,34 g (14,25 mmoles) de 3,5-di-tert-butyl-4-hydroxybenzaldéhyde et 1 cm³ de pipéridine. Chauffer au reflux sous agitation pendant 3 heures. Evaporer le benzène, purifier le produit brut obtenu par 2 passages successifs sur colonne de gel de silice en éluant par du chlorure de méthylène. Recueillir 1,70 g de poudre jaune correspondant au composé du titre.
Point de fusion : 175°C
Caractéristiques spectrales :

| | | |
|---|---|---|
| Infrarouge ν cm⁻¹ | ν C = O | 1 705 |
| | ν NH | 3 450 |
| | ν OH | 3 590 |

### EXEMPLES 21 A 23 :

En procédant comme dans l'exemple 20 mais en remplaçant la 1-(pyrid-2-yl)indolin-2-one par l'indolinone convenablement substituée, on obtient les composés des exemples suivants.
- **EXEMPLE 21**: **: 3-(3,5-DI-TERT-BUTYL-4-HYDROXYBENZYLIDENYL)-1-(2,4-DIMETHYL-PYRID-6-YL)-INDOLIN-2-ONE**
- **EXEMPLE 22**: **: 3-(3,5-DI-TERT-BUTYL-4-HYDROXYBENZYLIDENYL)-1-(6-METHOXY-PYRID-2-YL)-INDOLIN-2-ONE**
- **EXEMPLE 23**: **: 3-(3,5-DI-TERT-BUTYL-4-HYDROXYBENZYLIDENYL)-5-CHLORO-1-(PYRID-2-YL)-INDOLIN-2-ONE**
- **EXEMPLE 24**: **: 3-(3,5-DI-TERT-BUTYL-4-HYDROXYBENZYLIDENYL)-1-METHOXY-INDOLIN-2-ONE**

En procédant comme dans l'exemple 1, mais en remplaçant la 6-bromo-1-méthoxy-indolin-2-one par la 1-méthoxy-indolin-2-one, on obtient le composé du titre.
Point de fusion : 180 °C
Solvant de recristallisation : éther isopropylique
Caractéristiques spectrales :

| | | |
|---|---|---|
| Infrarouge ν cm⁻¹ | ν C = O | 1 715, 1 695 |
| | ν OH | 3 610 |
| | ν O - CH₃ | 2 870 |
| | ν C = C | 1 625, 1 610, 1 595 |

En procédant comme dans l'exemple 1, mais en remplaçant la 6-bromo-1-méthoxy-indolin-2-one par l'indolinone convenablement substituée, on obtient les composés des exemples suivants :
- **EXEMPLE 25**: **: 1-ACETYL-3-(3,5-DI-TERT-BUTYL-4-HYDROXYBENZYLIDENYL)-INDOLIN-2-ONE**
- Point de fusion: : 152°C
- **EXEMPLE 26**: **: 1-[3-(3,5-DI-TERT-BUTYL-4-HYDROXYBENZILIDENYL)-INDOLIN-2-ONE]ACETATE D'ETHYLE**
- **EXEMPLE 27**: **: ACIDE 1-[3-(3,5-DI-TERT-BUTYL-4-HYDROXYBENZILIDENYL)-INDOLIN-2-ONE]ACETIQUE**

### EXEMPLES 28 A 30 :

En soumettant les composés des exemples 1 à 3 au réactif de Lawesson, on obtient les composés des exemples suivants :
- **EXEMPLE 28**: **: 6-BROMO-3-(3,5-DI-TERT-BUTYL-4-HYDROXYBENZYLIDENYL)-1-METHOXY-INDOLIN-2-THIONE**
- **EXEMPLE 29**: **: 3-(3,5-DI-TERT-BUTYL-4-HYDROXYBENZYLIDENYL)-6-CHLORO-1-METHOXY-INDOLIN-2-THIONE**
- **EXEMPLE 30**: **: 3-(3,5-DI-TERT-BUTYL-4-HYDROXYBENZYLIDENYL)-1-(2,6-DICHLOROPHENYL)-INDOLIN-2-THIONE**
- E**XEMPLE 31**: **: 6-CHLORO-3-(3,5-Di-TERT-BUTYL-4-HYDROXYBENZYLIDENYL)-1-(2,6-DICHLOROPHENYL)-INDOLIN-2-ONE**

En procédant comme dans l'exemple 1, mais en remplaçant la 6-bromo-1-méthoxy-indolin-2-one par la 6-chloro-1-(2,6-dichlorophényl)-indolin-2-one, on obtient le composé du titre.

### Exemple A : ETUDE DE L'ACTIVITE ANTIPEROXYDANTE DES COMPOSES DE L'INVENTION

L'action des composés de l'invention, susceptibles de piéger les radicaux ·OH, a été étudiée d'une part, sur la peroxydation spontanée des lipides et d'autre part, sur la peroxydation induite par le système Fe²⁺ - ascorbate (10 µM - 250 µM), et ce sur des homogénats de cerveaux de rats.

Lors de la mesure de la peroxydation lipidique spontanée, les homogénats de cerveau de rats sont placés en présence ou en absence des composés à tester durant 60 min à 37°C. La réaction est arrêtée à 0°C et le dosage du malondialdéhyde est effectué à l'aide d'acide thiobarbiturique. La peroxydation lipidique est déterminée par les substances réagissant avec l'acide thiobarbiturique exprimées en nanomoles de malondialdéhyde.

Lors de la mesure de la peroxydation lipidique induite, la méthodologie est identique à celle précédemment décrite à l'exception de l'addition à l'homogénat du système inducteur de radicaux : Fe²⁺ - ascorbate. Les substances de référence sont le probucol et la vitamine E.

Les concentrations des composés testés inhibant de 50 % la peroxydation du substrat sont calculées.

Il est apparu que les composés de formule (I) utilisés selon l'invention possèdent une activité antiperoxydante particulièrement intense puisqu'ils possèdent une activité antipéroxydante nettement plus importante que le probucol et la vitamine E, qui est l'antioxydant naturel de l'organisme humain. Ce résultat remarquable se produit que la peroxydation soit spontanée ou induite par un système chimique.

### Exemple B : ETUDE DU POUVOIR PROTECTEUR DE L'OXYDATION DES LDL DES COMPOSES DE L'INVENTION

La capacité des composés de l'invention à diminuer les proportions de LDL oxydées a été mesurée de la façon suivante :
On réalise une incubation de 24 heures regroupant des LDL natives, un système Cu²⁺ générateur de radicaux libres et les composés à tester.
Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol.
Il apparaît clairement que les composés utilisés selon l'invention ont un pouvoir protecteur très important et significativement supérieur à celui du probucol ou aux dérivés du brevet US 5,124,347

### Exemple C : ETUDE DE L'ACTIVITE INHIBITRICE DES COMPOSES DE L'INVENTION SUR LA SYNTHESE DE PROSTANOIDES

### 1) ETUDE DE L'ACTIVITE INHIBITRICE SUR LA SYNTHESE DES PROSTANOIDES ISSUS DE LA CYCLOOXYGENASE

Le but de cette étude est de mesurer l'activité inhibitrice des composés de l'invention sur la sécrétion de prostaglandine E₂ (PGE₂), un des principaux prostanoïdes produits par la cyclooxygénase des granulocytes humains stimulés par l'ionophore calcique A23187.

### PROTOCOLE :

### - Isolement des granulocytes humains :

Du sang veineux humain provenant de donneurs sains n'ayant pas pris de médicaments depuis 2 semaines est prélevé dans des tubes en polypropylène contenant 1 volume d'anti-coagulant (acide citrique 2,73 %, citrate de sodium 4,48 %, glucose 2 %) pour 10 volumes de sang.

Dans l'heure suivant le prélèvement, du dextran à 6 % est ajouté au sang (0,3 cm³/cm³ de sang). Après une incubation de 30 mn à 37° C, le plasma riche en globules blancs est centrifugé à la vitesse de 100 g pendant 5 mn à 4° C.

Le culot est remis en suspension dans 3 cm³ de NH₄Cl à 0,83 % (pour lyser les globules rouges contaminants) et centrifugé à une vitesse de 100 g pendant 5 mn à 4° C.

Le culot riche en globules blancs mono- et polynuclés est récupéré dans 5 cm³ de tampon phosphate (pH 7,4) de composition suivante (en mM) : 137 : NaCl ; 2,68 : KCl ; 8,1 : Na₂HPO₄ ; 1,47 : KH₂PO₄ ; 0,9 : CaCl₂ ; 0,5 : MgCl₂ et déposé sur 3 cm³ d'une solution de ficoll type 400 à une densité de 1,077.

Après centrifugation à une vitesse de 420 g pendant 30 mn à 4° C, le culot riche en granulocytes est remis en suspension dans 5 cm³ de tampon phosphate et centrifugé à une vitesse de 100 g, 5 min à 4° C. Finalement, les granulocytes sont comptés et la densité est ajustée à 3 × 10⁶ cellules/cm³ de tampon phosphate.

### - Stimulation des granulocytes par l'ionophore calcique A23187 :

Les cellules (3 × 10⁶ cellules/cm³) sont préincubées à 37° C pendant 15 mn en absence ou en présence des produits à tester à la concentration désirée. Puis les cellules sont stimulées pendant 15 min à 37° C avec le A23187 à 5 × 10⁻⁶ M (solution mère à 10⁻² M dans le DMSO). Le taux basal est mesuré à partir de cellules ne recevant ni produits à tester, ni A23187.

La réaction est stoppée dans la glace et le surnageant est récupéré après centrifugation à une vitesse de 250 g pendant 5 min à 4° C.

### - Dosage de PGE₂ :

La quantité de PGE₂ produite est mesurée par un test radioimmunologique (RIA). Une gamme d'étalonnage est effectuée dans les mêmes conditions avec des concentrations communes de PGE₂.

### - Résultats :

Les composés de l'invention montrent une activité inhibitrice de la synthèse des prostanoïdes issus de la cyclooxygénase très supérieure à celle du probucol.

A titre d'exemple, à une concentration de 10⁻⁵ M, les composés de l'invention permettent une inhibition de la production de PGE₂ de 88 % alors que le probucol donne seulement une inhibition de l'ordre de 40 %.

### 2) ETUDE DE L'ACTIVITE INHIBITRICE SUR LA SYNTHESE DES PROSTANOIDES ISSUS DE LA LIPOXYGENASE

L'activité inhibitrice sur la synthèse des prostanoïdes des composés de l'invention est mesurée sur des cellules polynucléaires humaines lavées, en présence ou en absence du composé à tester, après activation de ces cellules par le calcium (ionophore calcique A 23187).

La production du principal prostanoïde, issu de la lipoxygénase, produit par les cellules polynucléaires humaines : le leucotriène B₄ (LTB₄) est mesurée par un test radioimmunologique.

Les composés de l'invention montrent donc une activité inhibitrice de la synthèse des prostanoïdes issus de la lipoxygénase très supérieure à celle du probucol.

A titre d'exemple, à une concentration de 10⁻⁵ M, les composés de l'invention permettent une inhibition de la production de LTB₄ supérieure à 95 % alors que le probucol donne seulement une inhibition de l'ordre de 40 %.

### CONCLUSION :

Les études 1 et 2 de l'exemple C montre que les composés de l'invention possèdent une intense activité inhibitrice sur la synthèse des prostanoïdes. Celle-ci est nettement supérieure à celle des composés du brevet US 5 124 347

### Exemple D : ETUDE DE L'ACTIVITE ANTIINFLAMMATOIRE DES COMPOSES DE L'INVENTION

### PRINCIPE :

Une inflammation expérimentale, de nature immunologique, est produite en injectant dans le coussinet plantaire de la patte du rat, un sérum anti-rat. La réaction inflammatoire se traduit par un oedème de la patte.

Le but de cette étude est de rechercher l'effet antiinflammatoire de composés de l'invention dans le modèle d'oedème d'origine immunologique chez le rat.
Les paramètres mesurés sont :
   - le volume de l'oedème,
   - la génération de prostaglandines E₂ (PGE₂),

### PROTOCOLE :

Les animaux utilisés dans cette étude sont des rats mâles Wistar de 350 à 400 g.

### - Animaux:

Un oedème localisé est produit au temps t₀ de l'étude par une injection intraplantaire de 0,1 cm³ de sérum de lapin anti-rat.

Des animaux témoins négatifs reçoivent une injection intraplantaire de 0,1 cm³ de liquide physiologique.

### - Traitement des animaux :

Les produits sont mis en suspension dans de la gomme arabique.

Une heure avant l'induction de l'oedème, les animaux reçoivent les produits, administrés per os, par sonde oesophagienne à une dose comprise entre 3 et 30 mg/kg.

Des animaux témoins positifs de même que les témoins négatifs reçoivent de la gomme arabique par sonde oesophagienne.

### - Mesure du volume de l'oedème :

L'oedème est caractérisé par l'augmentation du volume de la patte, déterminée à l'aide d'un pléthysmomètre à eau.

Une mesure initiale du volume de la patte est réalisée avant tout traitement.

Une autre mesure est réalisée 2 heures après l'induction de l'oedème.

### - Mesure de la PGE₂:

Les animaux sont sacrifiés juste après la mesure pléthysmométrique.

La région enflammée est prélevée et placée dans une solution à pH 3,0 contenant 33 % de CH₃CN.

Après homogénéisation, les PGE₂ sont extraites sur colonne éthyl C₂ (Amersham) et éluées avec du formate de méthyle.

Après évaporation sous azote, les PGE₂ sont solubilisées dans 200 µl de tampon phosphate.

Les concentrations de PGE₂ sont mesurées par analyse radioimmunologique.

### RESULTATS :

Il apparaît que les composés de l'invention, dès la dose de 3 mg/kg, permettent une inhibition très importante de volume de l'oedème provoqué et entraînent également une baisse significative de la production de la prostaglandine E₂, qui est un des médiateurs majeurs de l'inflammation.

### Exemple E : ETUDE DE LA TOXICITE AIGUE

La toxicité aiguë a été appréciée après administration orale à des lots de 3 souris (20 ± 2 grammes) de doses croissantes (0,1 - 0,25 - 0,50 - 0,75 - 1g/kg) de composés de l'invention. Les animaux ont été observés à intervalles réguliers au cours de la première journée et quotidiennement pendant les 2 semaines suivant le traitement.

Il apparaît que les composés de l'invention sont totalement atoxiques. Aucun décès n'est observé après une administration d'une dose de 1 g.kg⁻¹. On ne constate pas de troubles après administration de cette dose.

### Exemple F : COMPOSITION PHARMACEUTIQUE : COMPRIMES

Comprimés dosés à 15 mg de 6-chloro-3-(3,5-di-tert-butyl-4-hydroxybenzylidényl)-1-méthoxy-indolin-2-one
Formule de préparation pour 1000 comprimés :

| | |
|---|---|
| 6-chloro-3-(3,5-di-tert-butyl-4-hydroxybenzylidényl)-1-méthoxy-indolin-2-one | 15 g |
| Amidon de blé | 60g |
| Lactose | 45 g |
| Stéarate de magnésium | 0,5 g |
| Silice | 0,2 g |
| Hydroxypropylcellulose | 0,5 g |

## Revendications

1. Composés de formule (I) : 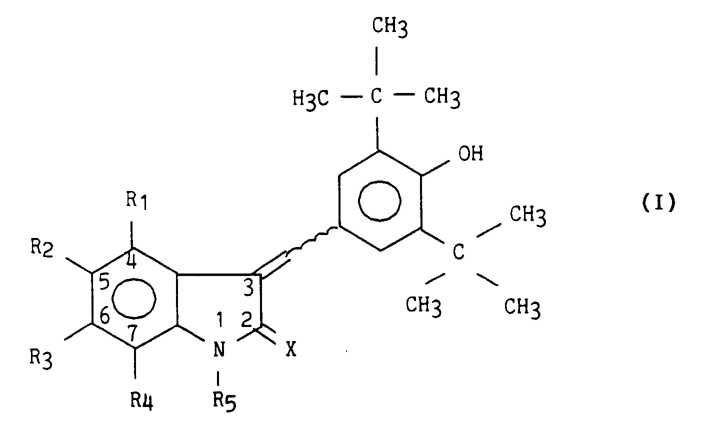 dans laquelle :
X représente un atome de soufre ou d'oxygène,
R₁, R₂, R₃, R₄, identiques ou différents, représentent chacun indépendamment l'un de l'autre :
- un atome d'hydrogène,
- ou un radical choisi parmi :
. halogène,
. hydroxyle,
. un groupement -E₁ ou 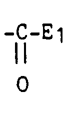 où E₁ représente un groupement alkyle inférieur, alcényle inférieur, alcynyle inférieur, ou alkoxy inférieur, le groupement E₁ pouvant être non substitué ou substitué par un ou plusieurs radicaux choisis parmi halogène, alkyl inférieur, alkoxy inférieur, alkylamino inférieur, et dialkylamino inférieur,
. et un groupement -(CH₂)ₙ-E₂ -O-(CH₂)ₙ-E₂, 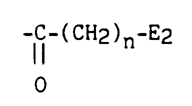 ou 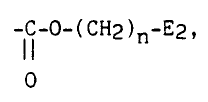 où n représente 0 ou un entier de 1 à 4 et où E₂ est choisi parmi :
- phényle ou naphtyle, non substitués ou substitués par un ou plusieurs radicaux choisi parmi halogène, hydroxyle, alkyle inférieur, alkoxy inférieur, et trifluorométhyle,
- et cycloalkyle de 3 à 8 atomes de carbone, non substitué ou substitué par un ou plusieurs radicaux choisis parmi halogène, oxo, alkyle inférieur, et alkoxy inférieur,
R₅ représente un radical choisi parmi :
- hydroxyle,
- un groupement -E₃ où E₃ représente un groupement acyle inférieur, alkoxy inférieur, ou un groupement -(CH₂)ₙ-CO-R₆ dans lequel n représente 0 ou un nombre entier de 1 à 6 et où R6 représente un radical hydroxyle ou alkoxy inférieur,
le groupement E3 pouvant être non substitué ou substitué par un ou plusieurs radicaux choisis parmi halogène, hydroxyle, alkyle inférieur et alkoxy inférieur,
- un groupement phényle ou -O-(CH₂)ₘ-phényle, où m représente 0 ou un nombre entier de 1 à 4, le noyau phényle pouvant être non substitué ou substitué par un ou plusieurs radicaux choisis parmi halogène, hydroxyle, alkyle inférieur, alkoxy inférieur, et trifluorométhyle,
- un groupement pyridyle ou -O-(CH₂)ₘ-pyridyle où m est tel que défini précédemment, le noyau pyridyle pouvant être non substitué ou substitué par un ou plusieurs radicaux choisis parmi halogène, hydroxyle, alkyle inférieur, et alkoxy inférieur,
- un groupement -(CH₂)ₘ-E₄, -O-(CH₂)ₘ-E₄, ou 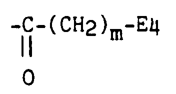 où m représente 0 ou un nombre entier de 1 à 4 et E₄ est un radical choisi parmi naphtyle, pyrimidinyle, thiényle, furyle, et pyrrolyle, E₄ étant non substitué ou substitué par un ou plusieurs radicaux choisis parmi halogène, hydroxyle, alkyle inférieur, alkoxy inférieur, et trifluorométhyle,
- et un goupement cycloalkyle ou cycloalkylalkyle inférieur, où le radical cycloalkyle contient de 3 à 8 atomes de carbone et est non substitué ou substitué par un ou plusieurs radicaux choisis parmi halogène, oxo, alkyle inférieur, et alkoxy inférieur,
étant entendu que, sauf précision contraire :
- les termes "alkyle inférieur", "alkoxy inférieur", et "acyle inférieur", désignent des groupements linéaires ou ramifiés contenant de 1 à 6 atomes de carbone,
- les termes "alcényle inférieur" et "alcynyle inférieur" représentent des groupements insaturés de 2 à 6 atomes de carbone,
leurs isomères Z et E, leurs isomères optiques, sous forme pure ou sous forme de mélange, et leurs sels d'addition à un acide ou une base pharmaceutiquement acceptable.

2. Composés de formule (I) selon la revendication 1, pour lesquels R₅ représente un groupement alcoxy inférieur, leurs isomères Z et E, leurs isomères optiques, sous forme pure ou sous forme de mélange, et leurs sels d'addition à un acide ou une base pharmaceutiquement acceptable.

3. Composés de formule (I) selon la revendication 1 pour lesquels R₁, R₂, R₃ et R₄ sont choisis parmi hydrogène, halogène, hydroxyle, alkyle inférieur, alcoxy inférieur, et trifluorométhyle, leurs isomères Z et E, leurs isomères optiques, sous forme pure ou sous forme de mélange, et leurs sels d'addition à un acide ou à une base pharmaceutiquement acceptable.

4. Composé selon la revendication 1 qui est la 6-chloro-3-(3,5-di-tert-butyl-4-hydroxybenzylidényl)-1-méthoxy-indolin-2-one et ses isomères Z et E.

5. Composé selon la revendication 1 qui est la 3-(3,5-di-tert-butyl-4-hydroxybenzylidényl)-1-(2,6-dichlorophényl)-indolin-2-one et ses isomères Z et E.

6. Composé selon la revendication 1 qui est la 3-(3,5-di-tert-butyl-4-hydroxybenzylidényl)-1-méthoxy-indolin-2-one, ses isomères Z et E et ses sels d'addition à un acide pharmaceutiquement acceptable.

7. Composé selon la revendication 1 qui est la 6-bromo-3-(3,5-di-tert-butyl-4-hydroxybenzylidényl)-1-méthoxy-indolin-2-one et ses isomères Z et E.

8. Composé selon la revendication 1 qui est la 3-(3,5-di-tert-butyl-4-hydroxybenzylidényl)-1-(pyrid-2-yl)-indolin-2-one, ses isomères Z et E et ses sels d'addition à un acide pharmaceutiquement acceptable.

9. Procédé de préparation des composés de formule (I) selon la revendication 1 caractérisé en ce que
on fait réagir un composé de formule (II) 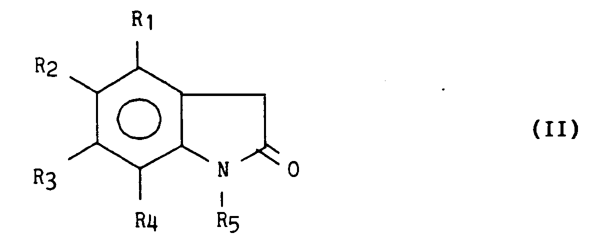 dans laquelle R₁, R₂, R₃, R₄, et R₅ sont tels que définis dans la revendication 1,
avec un composé de formule (III) 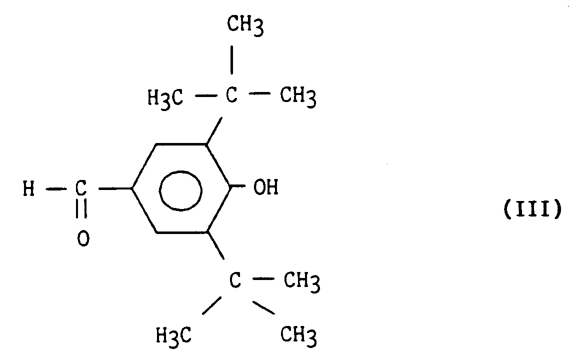 pour obtenir un composé de formule (I/a) 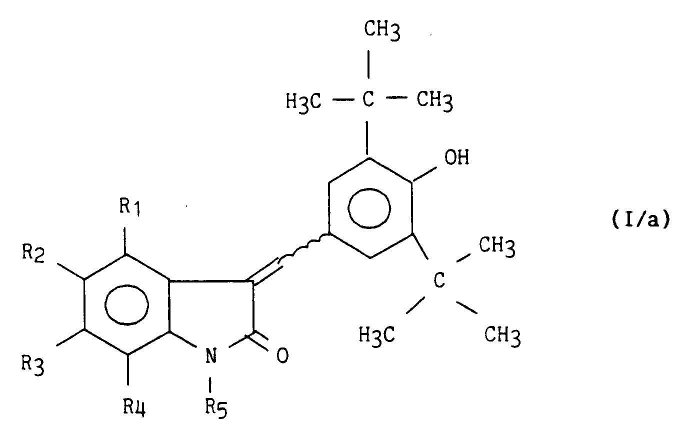 dans laquelle R₁, R₂, R₃, R₄ et R₅ sont tels que définis précédemment,
qui peut être soumis au réactif de Lawesson pour obtenir un composé de formule (I/b) 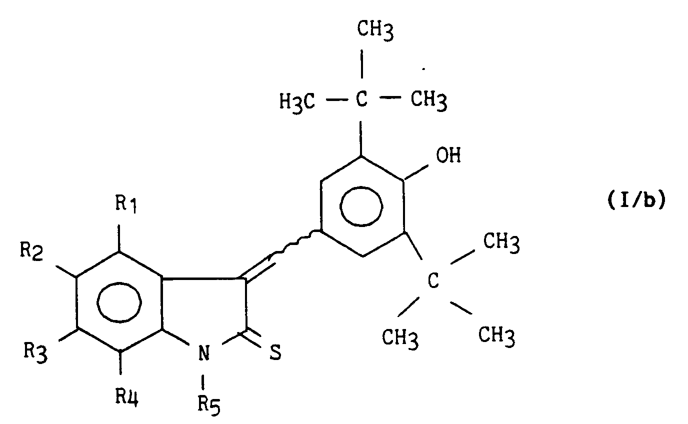 dans laquelle R₁, R₂, R₃, R₄ et R₅ sont tels que définis précédemment,
les composés de formule (I/a) et (I/b) formant l'ensemble des composés de formule (I) selon la revendication 1,
les composés de formule (I), selon la revendication 1, pouvant être :
- purifiés suivant une ou plusieurs méthodes de purification choisies parmi la cristallisation, la chromatographie sur colonne de silice, l'extraction, la filtration, et le passage sur charbon ou résine,
- séparés, le cas échéant, sous forme pure ou sous forme de mélange, en leurs éventuels isomères Z et E ou en leurs isomères optiques,
- ou salifiés par un acide ou une base pharmaceutiquement acceptable.

10. Composition pharmaceutique contenant comme principe actif au moins un composé selon la revendication 1, ou un de ses sels d'addition à un acide ou une base pharmaceutiquement acceptable, en combinaison avec un ou plusieurs excipients ou véhicules pharmaceutiquement acceptables.

11. Composition selon la revendication 10 utile dans le traitement ou la prévention des affections dues ou reliées à des phénomènes de peroxydation, à des dérèglements de la synthèse des prostanoïdes ou à des troubles de l'agrégation plaquettaire, dans le traitement des désordres ischémiques, des maladies inflammatoires, de la douleur, des maladies métaboliques, de l'athérome, de l'artériosclérose, des maladies respiratoires, de l'asthme, de l'emphysème, des maladies d'origine immunologique, du psoriasis, du lupus érythémateux, des réactions allergiques, du vieillissement cérébral ou périphérique, et dans la prévention et le traitement des dommages dus aux traumatismes chirurgicaux et à la reperfusion d'organes.

## Claims

1. Compounds of formula (I): 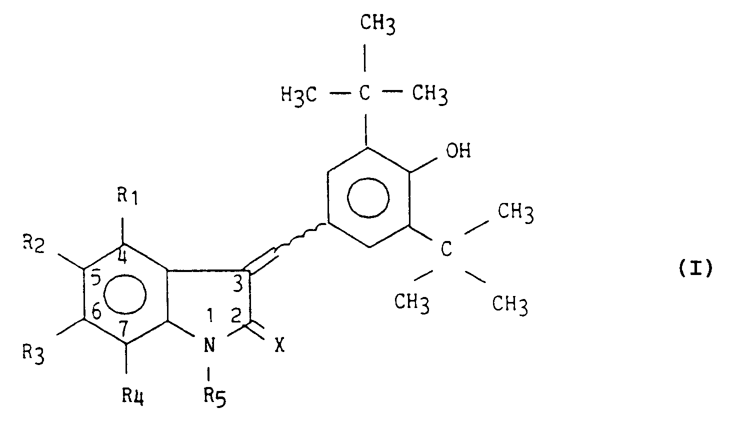 wherein :
X represents a sulphur or oxygen atom,
R₁, R₂, R₃ and R₄, which are the same or different, each represents, independently of the others :
- a hydrogen atom,
- or a radical selected from :
. halogen,
. hydroxy,
. a group -E₁ or 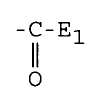 wherein E₁ represents a lower alkyl, lower alkenyl, lower alkynyl or lower alkoxy group, it being possible for the group E₁ to be unsubstituted or substituted by one or more radicals selected from halogen, lower alkyl, lower alkoxy, lower alkylamino and di-lower alkylamino,
. and a group -(CH₂)ₙ-E₂, -O-(CH₂)ₙ-E₂, 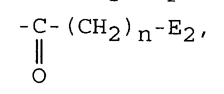 or 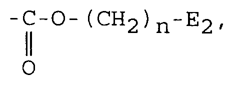 wherein n represents O or an integer of from 1 to 4 and wherein E₂ is selected from :
- phenyl and naphthyl, each of which is unsubstituted or substituted by one or more radicals selected from halogen, hydroxy, lower alkyl, lower alkoxy and trifluoromethyl,
- and cycloalkyl having from 3 to 8 carbon atoms, which is unsubstituted or substituted by one or more radicals selected from halogen, oxo, lower alkyl and lower alkoxy,
R₅ represents a radical selected from :
- hydroxy,
- a group -E₃ wherein E₃ represents a lower acyl group, a lower alkoxy group, or a -(CH₂)ₙ-CO-R₆ group wherein n represents O or an integer of from 1 to 6 and R₆ represents a hydroxy or lower alkoxy radical,
it being possible for the group E₃ to be unsubstituted or substituted by one or more radicals selected from halogen, hydroxy, lower alkyl and lower alkoxy,
- a phenyl or -O-(CH₂)ₘ-phenyl group wherein m represents O or an integer of from 1 to 4, it being possible for the phenyl nucleus to be unsubstituted or substituted by one or more radicals selected from halogen, hydroxy, lower alkyl, lower alkoxy and trifluoromethyl,
- a pyridyl or -O-(CH₂)ₘ-pyridyl group wherein m is as defined hereinbefore, it being possible for the pyridyl nucleus to be unsubstituted or substituted by one or more radicals selected from halogen, hydroxy, lower alkyl and lower alkoxy,
- a group -(CH₂)ₘ-E₄, -O-(CH₂)ₘ-E₄ or 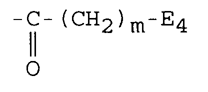 wherein m represents O or an integer of from 1 to 4 and E₄ is a radical selected from naphthyl, pyrimidinyl, thienyl, furyl and pyrrolyl, E₄ being unsubstituted or substituted by one or more radicals selected from halogen, hydroxy, lower alkyl, lower alkoxy and trifluoromethyl,
- and a cycloalkyl or cycloalkyl-lower alkyl group, wherein the cycloalkyl radical contains from 3 to 8 carbon atoms and is unsubstituted or substituted by one or more radicals selected from halogen, oxo, lower alkyl and lower alkoxy,
it being understood that, unless specified to the contrary :
- the terms "lower alkyl", "lower alkoxy" and "lower acyl" designate straight-chain or branched groups containing from 1 to 6 carbon atoms,
- and the terms "lower alkenyl" and "lower alkynyl" represent unsaturated groups having from 2 to 6 carbon atoms,
their Z and E isomers, their optical isomers, in pure form or in the form of a mixture, and addition salts thereof with a pharmaceutically acceptable acid or base.

2. Compounds of formula (I) according to claim 1, wherein R₅ represents a lower alkoxy group, their Z and E isomers, their optical isomers, in pure form or in the form of a mixture, and addition salts thereof with a pharmaceutically acceptable acid or base.

3. Compounds of formula (I) according to claim 1, wherein R₁, R₂, R₃ and R₄ are selected from hydrogen, halogen, hydroxy, lower alkyl, lower alkoxy and trifluoromethyl, their Z and E isomers, their optical isomers, in pure form or in the form of a mixture, and addition salts thereof with a pharmaceutically acceptable acid or base.

4. Compound according to claim 1, which is 6-chloro-3-(3,5-di-tert-butyl-4-hydroxybenzylidenyl)-1-methoxy-indolin-2-one and its Z and E isomers.

5. Compound according to claim 1, which is 3-(3,5-di-tert-butyl-4-hydroxybenzylidenyl)-1-(2,6-dichlorophenyl)-indolin-2-one and its Z and E isomers.

6. Compound according to claim 1, which is 3-(3,5-di-tert-butyl-4-hydroxybenzylidenyl)-1-methoxyindolin-2-one, its Z and E isomers, and addition salts thereof with a pharmaceutically acceptable acid.

7. Compound according to claim 1, which is 6-bromo-3-(3,5-di-tert-butyl-4-hydroxybenzylidenyl)-1-methoxy-indolin-2-one and its Z and E isomers.

8. Compound according to claim 1, which is 3-(3,5-di-tert-butyl-4-hydroxybenzylidenyl)-1-(pyrid-2-yl)indolin-2-one, its Z and E isomers, and addition salts thereof with a pharmaceutically acceptable acid.

9. Process for the preparation of compounds of formula (I) according to claim 1, characterised in that
a compound of formula (II) 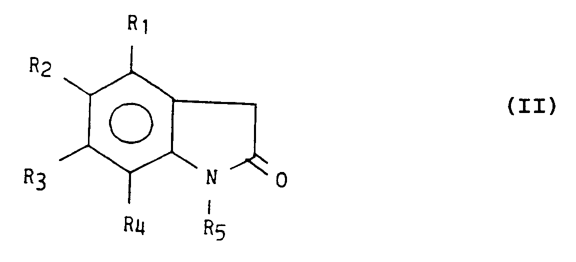 wherein R₁, R₂, R₃, R₄ and R₅ are as defined in claim 1, is reacted with
a compound of formula (III) 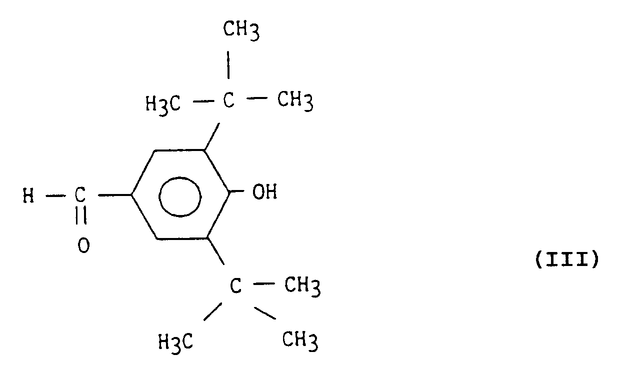 to obtain a compound of formula (I/a) 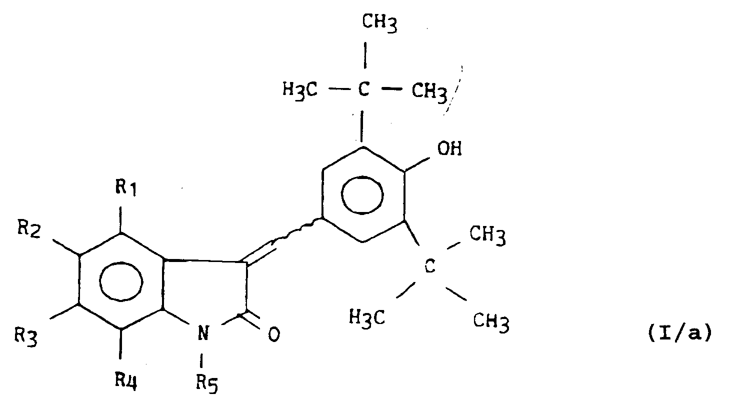 wherein R₁, R₂, R₃, R₄ and R₅ are as defined hereinbefore,
which may be subjected to the action of Lawesson's reagent to yield a compound of formula (I/b) 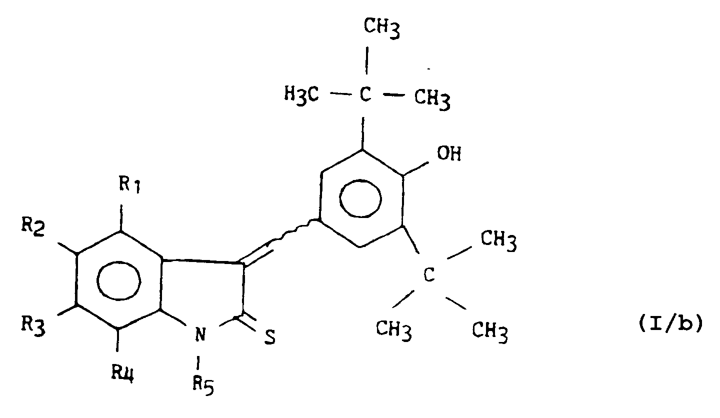 wherein R₁, R₂, R₃, R₄ and R₅ are as defined hereinbefore,
the compounds of formulae (I/a) and (I/b) forming the totality of the compounds of formula (I) according to claim 1,
it being possible for the compounds of formula (I) according to claim 1 to be:
- purified in accordance with one or more purification methods selected from crystallisation, chromatography on a silica column, extraction, filtration, and passage through charcoal or resin,
- separated, where appropriate, in pure form or in the form of a mixture, into their possible Z and E isomers or their optical isomers,
- or converted into salts with a pharmaceutically acceptable acid or base.

10. Pharmaceutical composition comprising as active ingredient at least one compound according to claim 1, or an addition salt thereof with a pharmaceutically acceptable acid or base, in combination with one or more pharmaceutically acceptable excipients or carriers.

11. Composition according to claim 10, which can be used in the treatment or prevention of disorders resulting from or associated with peroxidation phenomena, disturbances in prostanoid synthesis, or platelet aggregation disorders, in the treatment of ischaemic disorders, inflammatory disorders, pain, metabolic disorders, atheroma, arteriosclerosis, respiratory disorders, asthma, emphysema, disorders of immunological origin, psoriasis, lupus erythematosus, allergic reactions, cerebral or peripheral ageing, and in the prevention and treatment of damage resulting from surgical trauma and the perfusion of organs.

## Patentansprüche

1. Verbindungen der Formel (I): 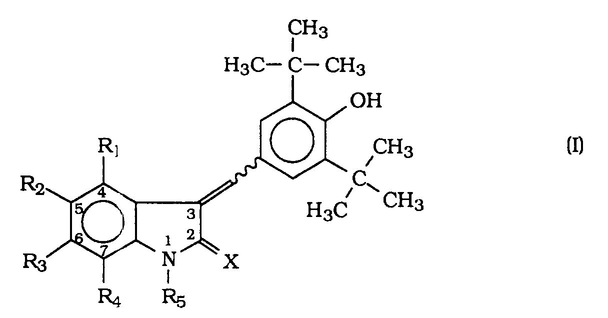 in der:
X ein Schwefel- oder Sauerstoffatom,
R₁, R₂, R₃ und R₄, die gleichartig oder verschieden sein können, jeweils unabhängig voneinander:
- ein Wasserstoffatom,
- oder eine Gruppe ausgewählt aus:
. Halogen,
. Hydroxyl,
. einer Gruppe -E₁ oder 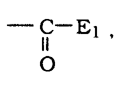 worin E₁ eine Niedrigalkylgruppe, Niedrigalkenylgruppe, Niedrigalkinylgruppe oder Niedrigalkoxygruppe darstellt, wobei die Gruppe E₁ unsubstituiert oder durch eine oder mehrere Gruppen ausgewählt aus Halogen, Niedrigalkyl, Niedrigalkoxy, Niedrigalkylamino und Niedrigdialkylamino substituiert sein kann,
. und einer Gruppe -(CH₂)ₙ-E₂, -O-(CH₂)ₙ-E₂, 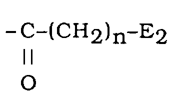 oder 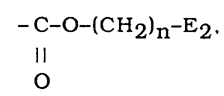 worin n 0 oder eine ganze Zahl mit einem Wert von 1 bis 4 darstellt und E₂ ausgewählt ist aus:
- Phenyl oder Naphthyl, die unsubstituiert oder durch eine oder mehrere Gruppen ausgewählt aus Halogen, Hydroxyl, Niedrigalkyl, Niedrigalkoxy und Trifluormethyl substituiert sein können,
- und Cycloalkyl mit 3 bis 8 Kohlenstoffatomen, welches unsubstituiert oder durch eine oder mehrere Gruppen ausgewählt aus Halogen, Oxo, Niedrigalkyl und Niedrigalkoxy substituiert sein kann,
R₅ eine Gruppe ausgewählt aus:
- Hydroxyl,
- einer Gruppe -E₃, worin E₃ eine Niedrigacylgruppe, Niedrigalkoxygruppe oder eine Gruppe -(CH₂)ₙ-CO-R₆ darstellt, worin n 0 oder eine ganze Zahl mit einem Wert von 1 bis 6 und R₆ eine Hydroxylgruppe oder eine Niedrigalkoxygruppe darstellen,
wobei die Gruppe E₃ unsubstituiert oder durch eine oder mehrere Gruppen ausgewählt aus Halogen, Hydroxyl, Niedrigalkyl und Niedrigalkoxy substituiert sein kann,
- einer Phenylgruppe oder -O-(CH₂)ₘ-Phenylgruppe, worin m 0 oder eine ganze Zahl mit einem Wert von 1 bis 4 darstellt und der Phenylkern unsubstituiert oder durch eine oder mehrere Gruppen ausgewählt aus Halogen, Hydroxyl, Niedrigalkyl, Niedrigalkoxy und Trifluormethyl substituiert sein kann,
- einer Pyridylgruppe oder -O-(CH₂)ₘ-Pyridylgruppe, worin m die oben angegebenen Bedeutungen besitzt, wobei der Pyridylkern unsubstituiert oder durch eine oder mehrere Gruppen ausgewählt aus Halogen, Hydroxyl, Niedrigalkyl und Niedrigalkoxy substituiert sein kann,
- einer Gruppe -(CH₂)ₘ-E₄, -O-(CH₂)ₘ-E₄ oder 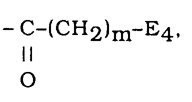 worin m 0 oder eine ganze Zahl mit einem Wert von 1 bis 4 darstellt und E₄ eine Gruppe ausgewählt aus Naphthyl, Pyrimidinyl, Thienyl, Furyl und Pyrrolyl darstellt und E₄ unsubstituiert oder durch eine oder mehrere Gruppen ausgewählt aus Halogen, Hydroxyl, Niedrigalkyl, Niedrigalkoxy und Trifluormethyl substituiert sein kann,
- und einer Cycloalkylgruppe oder Cycloalkylniedrigalkylgruppe, worin die Cycloalkylgruppe 3 bis 8 Kohlenstoffatome enthält und unsubstituiert oder durch eine oder mehrere Gruppen ausgewählt aus Halogen, Oxo, Niedrigalkyl und Niedrigalkoxy substituiert ist,
bedeuten, wobei es sich versteht, daß, wenn nichts anderes angegeben ist:
- die Begriffe "Niedrigalkyl", "Niedrigalkoxy" und "Niedrigacyl" für geradkettige oder verzweigte Gruppen mit 1 bis 6 Kohlenstoffatomen stehen,
- die Begriffe "Niedrigalkenyl" und "Niedrigalkinyl" für ungesättigte Gruppen mit 2 bis 6 Kohlenstoffatomen stehen,
deren Isomere Z und E, deren optische Isomere in reiner Form oder in Form einer Mischung und deren Additionssalze mit einer pharmazeutisch annehmbaren Säure oder Base.

2. Verbindungen der Formel (I) nach Anspruch 1, worin R₅ eine Niedrigalkoxygruppe darstellt, deren Isomere Z und E, deren optische Isomere in reiner Form oder in Form einer Mischung und deren Additionssalze mit einer pharmazeutisch annehmbaren Säure oder Base.

3. Verbindungen der Formel (I) nach Anspruch 1, worin R₁, R₂, R₃ und R₄ aus Wasserstoff, Halogen, Hydroxyl, Niedrigalkyl, Niedrigalkoxy und Trifluormethyl ausgewählt sind,
deren Isomere Z und E, deren optische Isomere in reiner Form oder in Form einer Mischung und deren Additionssalze mit einer pharmazeutisch annehmbaren Säure oder Base.

4. Verbindung nach Anspruch 1, nämlich 6-Chlor-3-(3,5-di-tert.-butyl-4-hydroxybenzylidenyl)-1-methoxy-indolin-2-on und dessen Isomere Z und E.

5. Verbindung nach Anspruch 1, nämlich 3-(3,5-Di-tert.-butyl-4-hydroxybenzylidenyl)-1-(2,6-dichlorphenyl)-indolin-2-on und dessen Isomere Z und E.

6. Verbindung nach Anspruch 1, nämlich 3-(3,5-Di-tert.-butyl-4-hydroxybenzylidenyl)-1-methoxy-indolin-2-on, dessen Isomere Z und E und dessen Additionssalze mit einer pharmazeutisch annehmbaren Säure.

7. Verbindung nach Anspruch 1, nämlich 6-Brom-3-(3,5-di-tert.-butyl-4-hydroxybenzylidenyl)-1-methoxy-indolin-2-on und dessen Isomere Z und E.

8. Verbindung nach Anspruch 1, nämlich 3-(3,5-Di-tert.-butyl-4-hydroxybenzylidenyl)-1-(pyrid-2-yl)-indolin-2-on, dessen Isomere Z und E und dessen Additionssalze mit einer pharmazeutisch annehmbaren Säure.

9. Verfahren zur Herstellung der Verbindungen der Formel (I) nach Anspruch 1, dadurch gekennzeichnet, daß man
eine Verbindung der Formel (II) 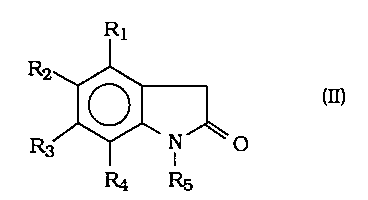 in der R₁, R₂, R₃, R₄ und R₅ die in Anspruch 1 angegebenen Bedeutungen besitzen, mit einer Verbindung der Formel (III) 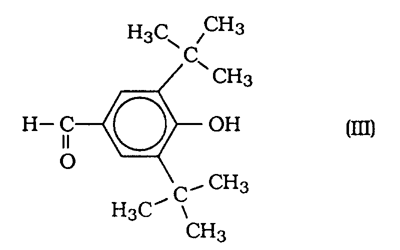 umsetzt zur Bildung einer Verbindung der Formel (I/a) 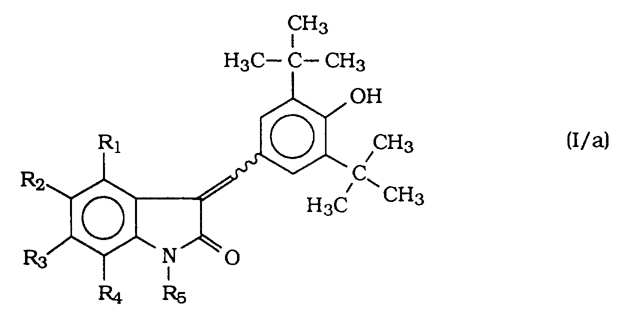 in der R₁, R₂, R₃, R₄ und R₅ die oben angegebenen Bedeutungen besitzen, welche der Einwirkung des Lawesson-Reagens unterzogen werden kann zur Bildung einer Verbindung der Formel (I/b) 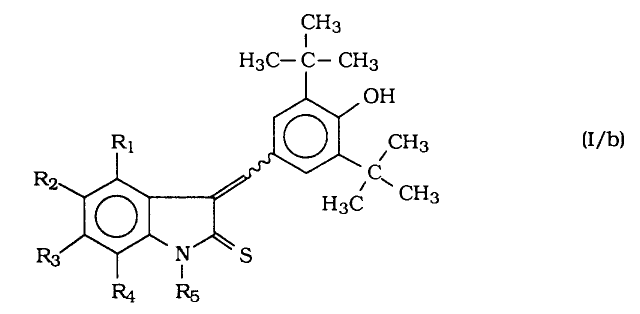 in der R₁, R₂, R₃, R₄ und R₅ die oben angegebenen Bedeutungen besitzen,
wobei die Verbindungen der Formeln (I/a) und (I/b) die Gesamtheit der Verbindungen der Formel (I) nach Anspruch 1 bilden,
wobei die Verbindungen der Formel (I) nach Anspruch 1:
- mit Hilfe eines oder mehrerer Reinigungsverfahren ausgewählt aus Kristallisation, Säulenchromatographie über Siliciumdioxid, Extraktion, Filtration und Überführung über Kohlenstoff oder ein Harz gereinigt werden können,
- gegebenenfalls in die Isomeren Z und E oder ihre optischen Isomeren in reiner Form oder in Form einer Mischung getrennt werden können, oder
- mit einer pharmazeutisch annehmbaren Säure oder Base in die Salze überführt werden können.

10. Pharmazeutische Zubereitung enthaltend als Wirkstoff mindestens eine Verbindung nach Anspruch 1 oder eines ihrer Additionssalze mit einer pharmazeutisch annehmbaren Säure oder Base in Kombination mit einem oder mehreren pharmazeutisch annehmbaren Trägermaterialien oder Bindemitteln.

11. Zubereitung nach Anspruch 10 zur Behandlung oder zur Vorbeugung von Erkrankungen, die eine Folge sind oder verknüpft sind mit Phänomenen der Peroxidation, von Mißsteuerungen der Synthese der Prostanoide oder von Störungen der Plättchenaggregation, bei der Behandlung von ischämischen Störungen, Entzündungserkrankungen, von Schmerz, Stoffwechselerkrankungen, Atheromen, Arteriosklerose, Atmungserkrankungen, Asthma, Emphysemen, Krankheiten immunologischen Ursprungs, der Psoriasis, Lupus erythematodes, allergischen Reaktionen, des zerebralen oder peripheren Alterns und bei der Vorbeugung und der Behandlung von Schäden als Folgen von chirurgischen Traumata und der Reperfusion von Organen.
